(19) **Europäisches Patentamt**

**European Patent Office**

**Office européen des brevets**

(11)     **EP 0 821 582 B1**

(12)                    **EUROPEAN PATENT SPECIFICATION**

(45) Date of publication and mention
of the grant of the patent:
**04.12.2002  Bulletin 2002/49**

(21) Application number: **96908487.0**

(22) Date of filing: **28.02.1996**

(51) Int Cl.⁷: **A61K 9/22**

(86) International application number:
**PCT/US96/02290**

(87) International publication number:
**WO 96/026718 (06.09.1996 Gazette 1996/40)**

(54) **CONTROLLED RELEASE TABLET**

TABLETTE MIT GESTEUERTER FREISETZUNG

COMPRIME A LIBERATION CONTROLEE

(84) Designated Contracting States:
**AT BE CH DE DK ES FR GB GR IE IT LI LU MC NL PT SE**

(30) Priority: **28.02.1995  US 395792**

(43) Date of publication of application:
**04.02.1998  Bulletin 1998/06**

(73) Proprietor: **Temple University of the Commonwealth System of Higher Education Philadelphia, PA 19122 (US)**

(72) Inventor: **KIM, Cherng-Ju Lansdale, PA 19446 (US)**

(74) Representative: **Schlich, George William et al Mathys & Squire European Patent Attorneys, 100 Gray's Inn Road London WC1X 8AL (GB)**

(56) References cited:
• **ADVANCE ACS ABSTRACTS, vol. 3, no. 3, 1 February 1995, XP002018917 C.J.KIM: "DRUG RELEASE FROM COMPRESSED HYDROPHILIC POLYOX-WSR TABLETS"**

**Description**

The present invention relates to sustained release tablets including any of a wide array of pharmaceutical products and an excipient.

Many attempts have been made in the pharmaceutical art to provide a composition which would enable the production of sustained release tablets. These attempts have produced compositions which are able to release pharmaceutical agents over a long period of time. However, such release has not been constant. Rather, prior art sustained release compositions generally release a disproportionate amount of their pharmaceutical agent quickly after ingestion by a patient. This results in a quick spike in the level of medication in the patient's bloodstream. Such a delivery method may be desirable where the pharmaceutical is intended to alleviate particular symptoms of a patient. However, there are many instances where a spike in the medication level in a patient is undesirable, as where pharmaceuticals are used to treat a chronic condition. Further, extended release of biologically active drugs is highly desirable for drugs that have characteristically short half lives.

Monolithic systems, composed of hydrophobic polymers and other excipients, are commonly used for extended release dosage forms because they are not costly or difficult to produce. However, such systems often provide a square-root-of-time (sqrt) kinetics (i.e. the amount of pharmaceutical agent released is proportional to the square root of the time since the drug was ingested). There are several ways to improve the release kinetics of extended release dosage forms. Incorporation of hydrophilic polymers into monolithic matrices modifies square root kinetics due to the swelling of the polymer. In particular, monolithic matrix systems controlled by the swelling/erosion processes of hydrophilic polymers can improve the pharmaceutical agent kinetics. However, even in these systems, the release rate of the pharmaceutical agent generally varies significantly with time.

Hydrophilic polymers alone have also been investigated for controlled drug release. The hydrophilic polymers such as hydroxypropylmethyl cellulose (HPMC) and polyvinylalcohol (PVA), which form gels upon contact with water and erode slowly, have been utilized for oral drug delivery systems. Cross-linked poly(ethylene oxide) has also been investigated for controlled drug delivery. However, attempts to use these materials to produce a constant, controlled, sustained drug release have thus far proven ineffective.

CJ Kim (Advance ACS Abstracts, volume 3, No. 3, page 107) discloses controlled release tablets comprising polyethylene oxide (PEO), a drug and magnesium stearate and states that drug release kinetics are influenced by the molecular weight of the PEO.

Ford et al. (Int J. Pham., 1985, vol 24, pp 327-338 and 339-350) discloses controlled release tablets comprising a drug, magnesium stearate and HPMC.

US-A-4,792,452(Howard) also disclose a controlled release tablet comprising a drug, magnesium stearate and HPMC.

Hansson et al. (J. Pharmaceutical Science, 1988, vol 77, No. 4, pp 322-324) discloses a HPMC - based tablet wherein the tablet has a central aperture.

US-A-4,816,262 (McMullen) discloses a controlled release tablet of unspecified composition having a central aperture.

The present invention provides a controlled release tablet including a pharmaceutical agent and excipient. The excipient includes at least about 50% of a water swellable polymer and a lubricant. The water swellable polymer is chosen such that the swellable rate of the polymer is equal to the dissolution rate of the swollen polymer. The lubricant is generally present in an amount of up to 3% of the excipient as a whole. The excipient may also include such other ingredients as diluents, fillers, binders and other pharmacologically inactive compounds. Further, the polymer and pharmaceutical agent are complementary. In selecting a water swellable polymer, the polymer should be chosen with the pharmaceutical agent in mind such that the tablet will be fully dissolved at the same time that the last of the pharmaceutical agent is released.

The preferred water swellable polymer is polyethylene oxide. The pharmaceutical agent is released from the tablet at a rate which can be expressed by $M_t/M_T = kt^n$ where t is time, $M_t$ is the amount of the pharmaceutical agent which has been released at time t, $M_T$ is the total amount of the pharmaceutical agent contained in the tablet, k is a constant, and n is the release kinetics exponent and has a value between about 0.89 and 1.0.

Thus the invention may provide a controlled release tablet that exhibits zero order releasing kinetics for the pharmaceutical agent when the tablet is swollen.

Figure 1 is a graph of the gel thickness and the tablet diameter versus time for various molecular weights of PEO polymer.

Figure 2 is a graph of the fractional release of theophylline from PEO tablets versus time, for various molecular weights of PEO polymer.

Figure 3 is a graph of the fractional release of theophylline from PEO tablets versus time at varying pH levels.

Figure 4 is a graph of the fractional release of theophylline and diltiazem hydrochloride from $0.9 \times 10^6$ molecular weight PEO tablets versus time for varying drug loadings.

Figure 5 is a graph of the fractional release of theophylline and diltiazem hydrochloride from $2.0 \times 10^6$ molecular weight PEO tablets versus time for varying drug loadings.

Figure 6 is a graph of the fractional release of theophylline from $0.9 \times 10^6$ molecular weight PEO tablets versus time for various rates of agitation.

Figure 7 is a graph of the release exponent (n) versus solubility of the drug for tablets of PEO of various molecular weights.

Figure 8 is a graph of the fractional release of various drugs versus time from tablets of $0.9 \times 10^6$ molecular weight PEO.

Figure 9 is a graph of the fractional release of various drugs versus time from tablets of $2.0 \times 10^6$ molecular weight PEO.

Figure 10 is a graph of the fractional release of various drugs versus time from tablets of $4.0 \times 10^6$ molecular weight PEO.

Figure 11 is a graph of the fractional release of theophylline versus time from tablets formed from hydroxypropylmethylcellulose of varying viscosity.

Figure 12 is a graph of the fractional release of theophylline from tablets of PEO having a molecular weight of $4.0 \times 10^6$ and from tablets of HPMC having a viscosity of 1500 - 1800 centipoise and having a hole through the center thereof.

Figure 13 is a graph of the fractional release from Procardia XL tablets having 30 mg and 60 mg of drug per tablet.

Figure 14 is a graph of the fractional release of nifedipine from tablets of PEO having various molecular weights having 10 percent nifedipine.

Figure 15 is a graph of the fractional release of nifedipine from tablets of PEO having a molecular weight of $4.0 \times 10^6$ and having various amounts of polyethylene glycol added thereto.

Figure 16 is a graph of the fractional release of nifedipine from tablets of PEO having a molecular weight of $4.0 \times 10^6$ and having various amounts of polyethylene glycol added thereto, compared to the fractional release of nifedipine from a Procardia XL tablets having 30 mg drug per tablet.

Figure 17 is a graph of the fractional release of nifedipine from tablets of PEO having a molecular weight of $4.0 \times 10^6$ having 20 percent nifeipine and having various amounts of polyethylene glycol added thereto.

Figure 18 is a graph of the fractional release of nifedipine from tablets of PEO having a molecular weight of $4.0 \times 10^6$ and having various amounts of polyethylene glycol added thereto, compared to the fractional release of nifedipine from Procardia XL tablets having 60 mg drug per tablet.

Figure 19 is a graph of the fractional release from Procardia XL tablets having 30 mg and 60 mg of drug per tablet compared to the fractional release of nifedipine from tablets of PEO having a molecular weight of $4.0 \times 10^6$ and having various amounts of nifedipine therein.

Figure 20 is a graph of the fractional release of nifedipine from tablets of PEO having a molecular weight of $4.0 \times 10^6$ and 10 percent nifdipine per tablet.

The excipients of the present invention have been formulated to allow controlled sustained release of any one of a number of pharmaceutical agents. The excipients of the present invention generally comprise a hydrophilic polymer and a lubricant. Other pharmacologically inactive materials can also be added. The excipients can be mixed with a wide range of medications and directly compressed into solid dosage tablets.

The following drugs with which the present invention may be used are set forth as examples only. Any suitable drug may be used with the present invention, including nifedipine, diclofenac NA, salicylic acid, theophylline anhydrous, sulfapyridine, sulfadrazine, propranolol hydrochloride, and diltiazem hydrochloride. A preferred pharmaceutical agent of the invention is diltiazem hydrochloride. The pharmaceutical agent is preferably present in an amount of from 10% to 40% by weight of the tablet. The tablets of the invention comprise a solubilizing agent. The solubilizing agent may be selected from the group consisting of polyethylene glycol, cyclodextrins, hydroxypropylcyclodextrins and polyvinylpyrrolidone (PVP). Preferably the solubilizing agent is present in an amount up to 150% of said excipients. Polyethylene glycol is generally used to increase the solubility of relatively insoluble drugs to encourage absorption by the body. The polymers set forth herein may be compressed into tablets directly. Alternatively, diluents, binders, filler or other typical compounding agents may be added to adjust the properties of the final tablets. Particularly, but without limitation, the following compounds may be included: lactose, dextrose, sucrose and calcium phosphate. The diluent can be present in an amount of up to 50% by weight of the excipient.

Specifically, the polymeric materials useful in the present invention will depend to a certain extent on the pharmaceutical agent chosen to be administered. Polymers of choice include uncrosslinked poly(ethylene oxide) (PEO) of varying molecular weight. PEO is available on the basis of molecular weight. Mixtures of various molecular weights of PEO may be used in the present invention. Preferably, the PEO has an average molecular weight of 900,000 to 4,000,000.

During the release of drugs from hydrophilic matrices of PEO two mechanistic phenomena take place: the swelling and the erosion of the polymer. The release kinetics of the drug from the tablet are dependent upon the relative magnitude of the rate of polymer swelling at the moving rubbbery/glassy front and the rate of polymer erosion at the swollen polymer/dissolution medium front. It is most preferable to attain the synchronization of the velocities of the swelling front and the erosion front in order to achieve zero-order release kinetics hydrophilic polymer matrices.

The release kinetics for the release of a pharmaceutical agent from a tablet can be approximated by the following equation:

$$(1) \qquad M_t/M_T = kt^n$$

where t is time,

$M_t$ is the amount of the pharmaceutical agent which has been released at time t,

$M_T$ is the total amount of the pharmaceutical agent contained in the tablet,

k is a constant, and

n is the release kinetics exponent.
This equation is valid so long as n remains nearly constant. When n is about equal to one, the release of the pharmaceutical agent from the tablet has zero-order kinetics. The amount of pharmaceutical agent released is then directly proportional to the time.

Where the swelling process of the polymer chosen for the excipient is the primary process controlling the drug release (compared to erosion of the swollen polymer), non-zero order release kinetics result. Generally, these release kinetics dictate a value of n approaching 0.5, leading to square-root Fickian-type release kinetics. Accordingly, a polymer must be chosen which will swell and erode readily, to allow erosion resulting in zero-order release kinetics.

Drug release from uncross-linked low molecular weight PEO of MW (molecular weight) equal to about $0.6 \times 10^6$ (laminated films) ensures a constant release rate by achieving synchronized gel thickness. That is, the rate of swelling of the polymer is approximately equal to the rate of erosion of the swollen polymer. On the other hand, the drug release from the high molecular weight PEO of MW = $4\times10^6$ is predominantly controlled by the swelling of the polymer rather than the erosion of the polymer. This can result in a non-constant release induced by the diffusion of drugs through the swollen gel layer, especially in high solubility drugs.

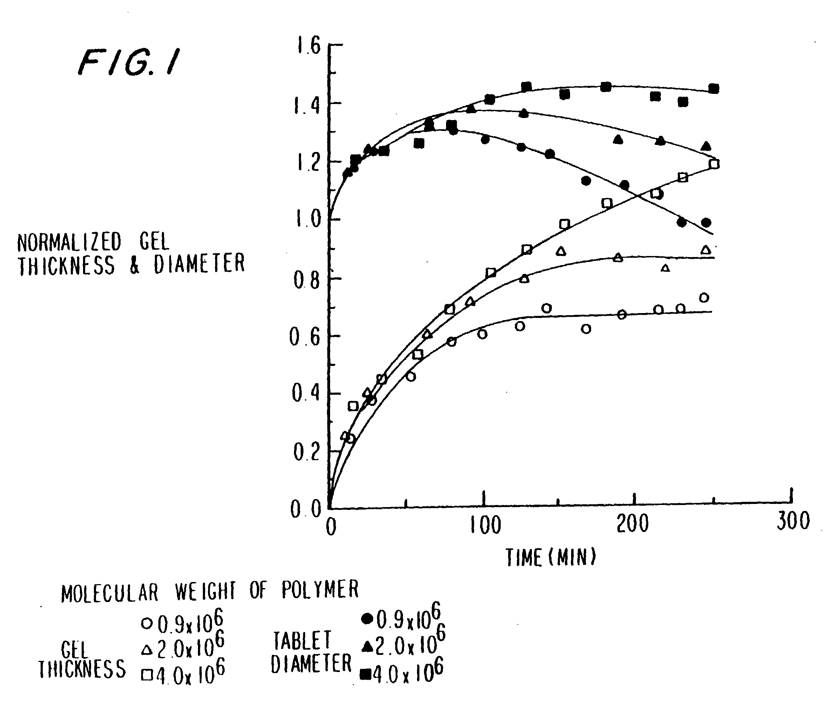

Drug release from PEO tablets is governed by swelling/drug diffusion or swelling/polymer erosion depending upon the characteristics of the different molecular weights of the hydrophilic polymers. In order to distinguish between the two mechanisms, swelling experiments involving drug-free tablets of PEO of MWs = $0.9\times10^6$, $2\times10^6$, and $4\times10^6$ were carried but although PEO with a molecular weight of up to $8\times10^6$ can also be used with more insoluble drugs. The swollen gel thickness and outer diameter of a drug-free PEO tablet are shown in Figure 1. Figure 1 shows that a constant gel thickness is obtained with the PEO tablets of MWs=$0.9\times10^6$ and $2\times10^6$, whereas the swollen gel thickness from the PEO tablet of $4\times10^6$ keeps increasing with time.

As can be seen from Figure 1, in the early time of the swelling/erosion process, the overall size of the tablet along with the gel layer increases rapidly due to the immediate swelling of the PEO tablet. The swelling rate of the polymer equals the dissolution rate of the swollen polymer gel, resulting in the synchronization of the gel layer thickness.

The release kinetics of the tablet are also dictated by the pharmaceutical agent itself. A drug which is highly soluble will tend to be released faster than drugs which have low solubility. Where a drug has high solubility, polymer swelling and erosion must take place rapidly to maintain a zero order release kinetics. If the swelling and erosion take place too slowly, the swelling process of the polymer is the primary process controlling the drug release (since the drug will diffuse from the swollen polymer before the polymer erodes). In this situation, non-zero order release kinetics result. As a result, the administration of a highly soluble pharmaceutical agent requires a relatively rapidly swelling and eroding excipient. To use such a material to produce a tablet which will last for 24 hours can require a large pill.

To overcome this difficulty, a doughnut-shaped tablet with a hole through the middle can be used with a less rapidly swelling and eroding polymer. With such a tablet, the surface area of the tablet increases as the tablet erodes. This exposes more polymer, resulting in more polymer swelling and erosion as the tablet shrinks in size. The type of tablet can also be used with very highly soluble pharmaceutical agents to maintain zero order release kinetics. Thus the invention can be in the form of a tablet wherein the tablet has an aperture therethrough.

Conversely, the delivery of an insoluble pharmaceutical agent is dictated only by the erosion of the swollen polymer layer, since there will be no diffusion and dissolution of the pharmaceutical agent out of the swollen polymer matrix. Accordingly, relatively slow swelling and eroding polymers can be used. In this case, if a sufficiently slow polymer is used, a tablet can be made very small with a higher concentration of pharmaceutical agent than would be used in a normal-sized tablet. This would result in a very small but very effective sustained release tablet.

EXAMPLE

POLYOX-WSR NFs (non-crosslinked polyethylene oxide) of average molecular weight of $0.9\times10^6$, $2\times10^6$, and $4\times10^6$ was obtained from Union Carbide Corporation (Danbury, Connecticut). Theophylline anhydrous U.S.P and magnesium stearate U.S. P were obtained from Amend Co. (Irving, New Jersey) and Mallinckrodt Chemical (Jersey City, New Jersey), respectively. Diltiazem HCI was obtained from Sigma Chemical Co. (St. Louis, Missouri). Hydroxypropylmethylcellulose was obtained from Dow Chemical (Midland, Michigan) and Shin-Etsu Chemical Co., Ltd. (Tokyo, Japan). The materials were used as received.

The ingredients (PEO, drug, and magnesium stearate) were mixed and then tableted using a single punch tablet machine (Stokes Model F, Philadelphia, Pennsylvania). A set of tablet punches with flat surfaces were used to prepare tablets with an 8.1 mm diameter. They were charged with the formulation, and the compression was exerted by turning over the machine by hand. The hardness of the resulting tablets was 5.5 to 7.0 Kp (Schleuniger Model 2E/106, Switzerland). The resulting tablets weighed about 160 mg.

In vitro release of drugs from the different formulation tablets was carried out by using the USP basket procedure in distilled/deionized water at a stirring rate of 100 rpm and 37°C, if not otherwise stated. Theophylline and diltiazem HCl were chosen as model drugs. The release of theophylline and diltiazem HCl was assayed by a HP8252A (Hewlett Packard Corporation) diode-array spectrophotometer at 244 nm and 266 nm, respectively. The release kinetic data (up to 60% release) were treated by equation (1) above. After this point, frequently n was no longer constant, and therefore equation (1) did not apply.

According to the criteria for release kinetics from a swellable matrix (cylindrical shape), zero-order and anomalous transport kinetics are represented by $0.89 < n < 1.0$ and $0.45 < n < 0.89$, respectively. A linear regression analysis (Microsoft Excel 5.0) of the logarithmic form of equation (1) was carried out with $R^2 \geq 0.99$.

Both circular faces of the cylindrical tablet were covered with two transparent Mylar sheets, fixed onto the bases via an epoxy glue. The covered tablet was then introduced into water as a dissolution medium. The tablet was withdrawn periodically, and the moving boundaries both at the glassy/rubbery interface and at the matrix/dissolution medium interface were measured via a stereo microscope (Olympus SZ60) with a measuring device attachment. The tablet was then returned to the bath.

As can be seen from Figure 1, the synchronization of the gel layer thickness takes place earlier with the low molecular weight PEO and shortly after the outer diameter starts becoming smaller. The higher the molecular weight, the later the synchronization takes place. However, for a higher molecular weight PEO tablet ($4x10^6$) the synchronization of the gel layer did not occur and the gel layer thickness increased until the entire tablet was in gel form. After the front meets at the core of tablets, the swollen PEO gel lasts a much longer time than other swellable/erodible polymers such as PMMA/MAA and PVA. The swollen PEO gel layer can last 2 to 4 hours depending upon the molecular weight of the PEO. As a result of this long residence time of the gel layer, it is expected that the release kinetics are governed by the erosion/drug diffusion process after the swelling front meets at the core of the tablet.

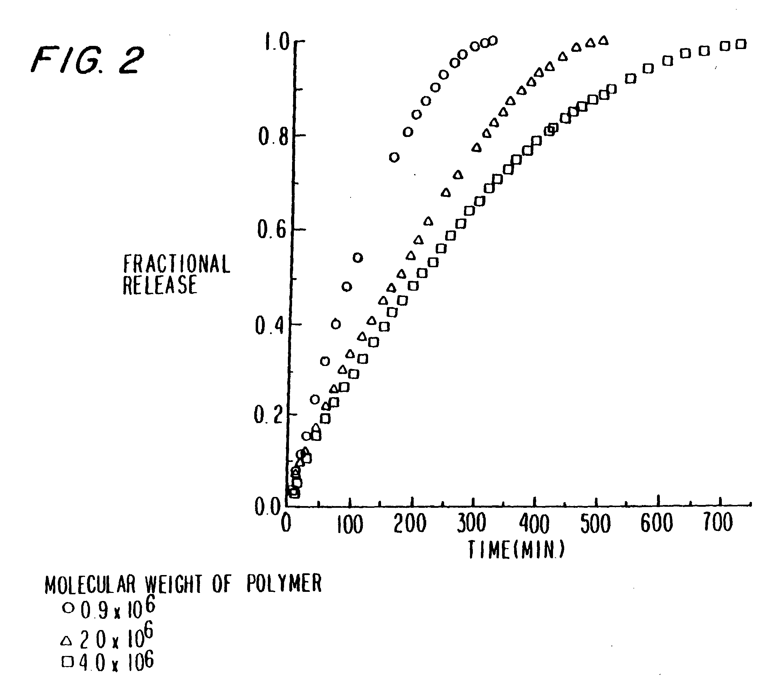

Figure 2 shows the effect of PEO molecular weight on drug release of theophylline from tablets. The rate of dissolution (erosion) of hydrophilic polymers decreases with increasing molecular weight. This causes different molecular weights of PEO to have different values for the release exponent (n). For the release of theophylline from PEO tablets, the value of the release exponent (n) was 0.99 ($\pm$0.024), 0.80 ($\pm$0.004), and 0.83 ($\pm$0.019) for tablets formed from PEO having molecular weights of $0.9x10^6$, $2x10^6$, and $4x10^6$, respectively. The lower molecular weight PEO (MW=$0.9x10^6$) tablet exhibits a constant release rate, whereas PEO tablets having molecular weights of $2x10^6$ and $4x10^6$ may vary, depending on the solubility of the drug used.

Accordingly, the synchronized gel thickness plays an important role on the release kinetics at the early time of drug release. As the molecular weight of the polymer increases, the dissolution rate of the polymer decreases compared to the swelling rate of the polymer. The "dissolution rate" and the "erosion rate" are used here interchangeably. In actuality, the decrease in the size of the swollen gel layer is due both to erosion and dissolution. Due to the low dissolution rate, the thickness of the polymer layer increases. Drug diffusion through the swollen gel layer then becomes the foremost process controlling the release mechanism. With high molecular weight (or high viscosity grade) polymers of PEO and HPMC, the swelling of the polymer is the dominant step in controlling release kinetics over the erosion of swollen polymer. With low molecular weight polymers of PEO and HPMC, the erosion of the polymer is the rate determining step for the release kinetics. However, the degree of swelling of PEO is higher than HPMC resulting in more favorable kinetics for PEO than for HPMC.

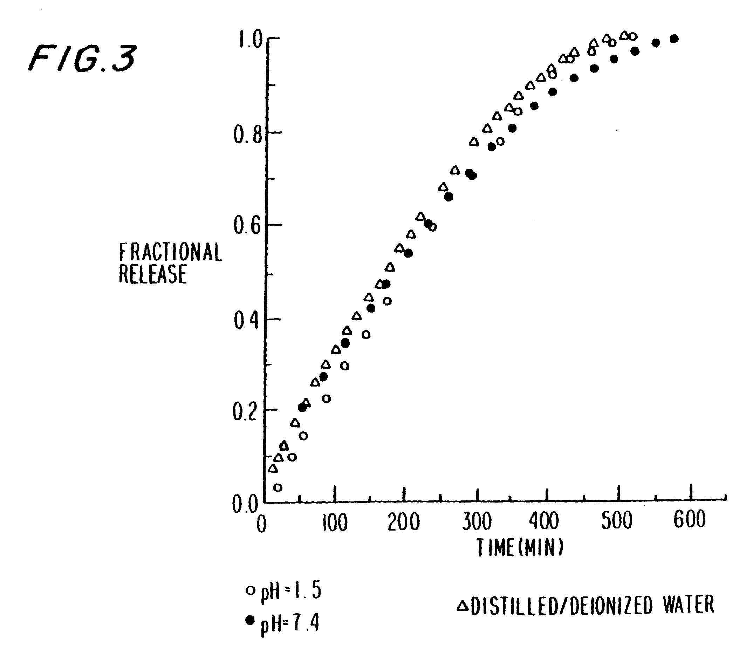

The release of theophylline from PEO tablets is not significantly influenced by the pH variation of the dissolution medium, as shown in Figure 3. Therefore PEO may be a good candidate for oral drug delivery systems because oral dosage forms travel along the gastrointestinal tract in which the pH changes from an acidic environment (pH < 2) in the stomach to a weak base (pH about 8) in the intestines.

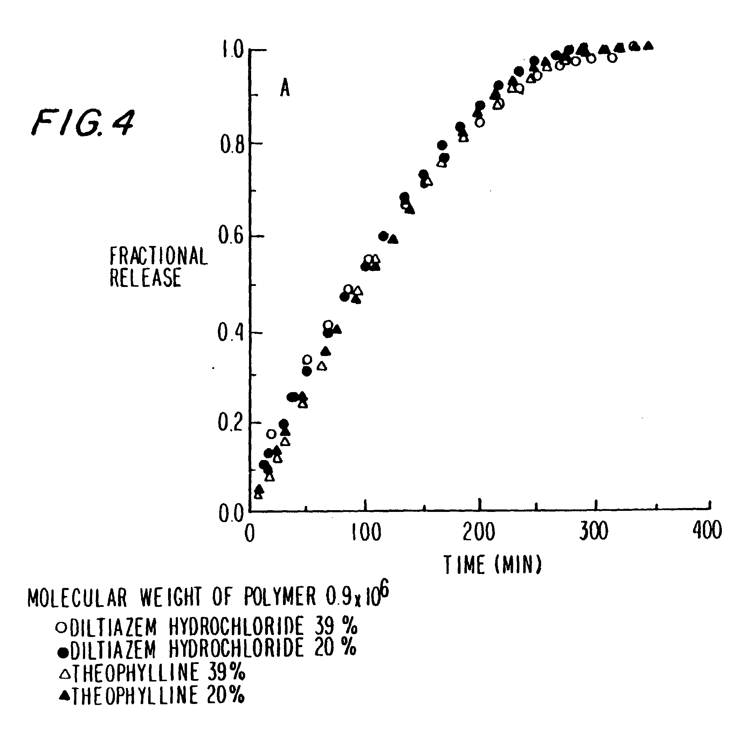

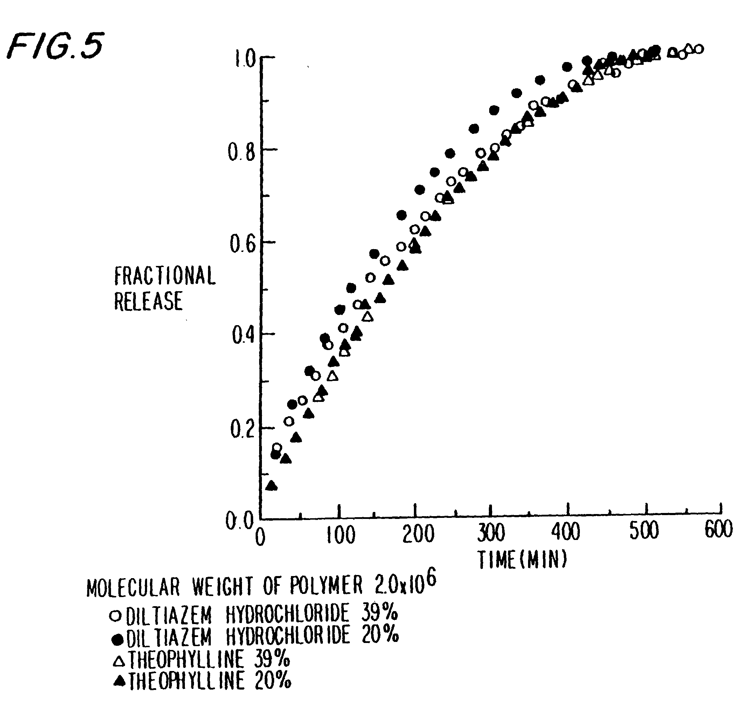

PEO tablets having varying loadings of pharmaceutical agents were tested to determine the effect of loading on the release kinetics. The results are shown in Figures 4 and 5. No significant difference in release rate was observed with the different drug loadings in PEO tablets.

The release kinetics of PEO tablets of MW=$0.9x10^6$ remain unchanged with the exponent n ranging from 0.90 ($\pm$0.016) to 0.99 ($\pm$0.024) even though the drug loading is increased. Even for PEO tablets of MW=$2x10^6$, the release profiles of theophylline from 20% and 39% loaded tablets are superimposed with the exponent n ranging from 0.80 ($\pm$0.004) to 0.82 ($\pm$0.007). In these cases, the diffusion of theophylline (< 1.0% solubility in water) through the swollen gel layer does not play an important role in the release mechanism in PEO tablets. Rather it is the rate of erosion of the gel layer which dictates the rate of release of the drug.

The diffusion of water into the matrices is not greatly facilitated by the water uptake of drugs but rather by the water uptake of the hydrophilic polymer. This demonstrates that the swelling/erosion of the polymer is the controlling variable rather than the diffusion of drugs through the swollen gel layer. This may not be true when a highly water-soluble drug (diltiazem HCl, solubility >50%) is employed. Figures 4 and 5 show the effect of drug solubility on the release of drugs from PEO tablets. With a low molecular weight PEO, the solubility of the drug does not markedly affect the release of drugs from PEO tablets as may occur with other hydrophilic polymers.

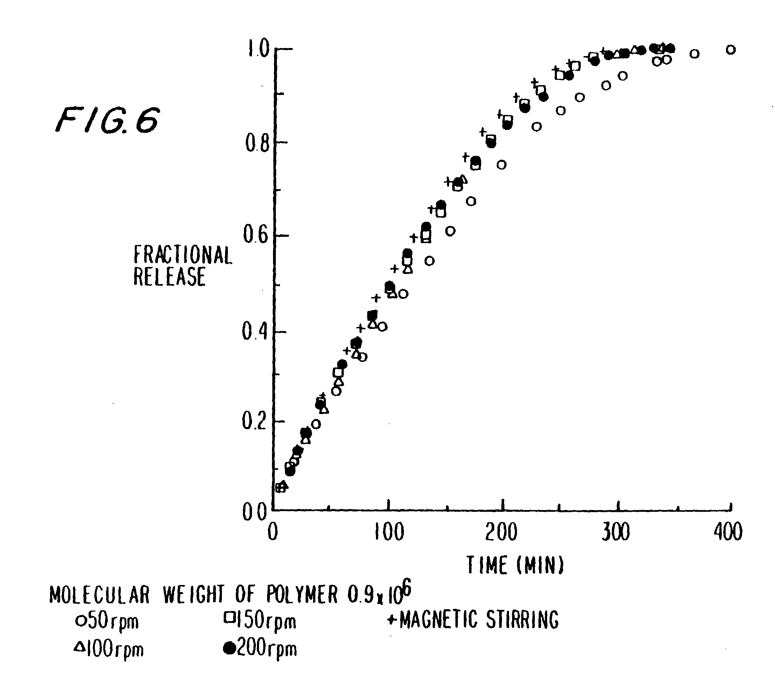

PEO tablets were also tested to determine the effect of stirring on the release of a drug from the tablet. The results are shown in Figure 6. As may be seen from Figure 6, the rate of release of the drug from the PEO tablet increased as stirring was increased with an increase in the stirring rate from 50 to 100 rpm. However, above 100 rpm, the rate of release of the drug was insensitive to the amount of stirring. The insensitivity of PEO tablets to the stirring rate also makes PEO a good candidate for oral dosage forms since the release rate will not vary as the tablet is subjected to turbulence in the stomach and intestine.

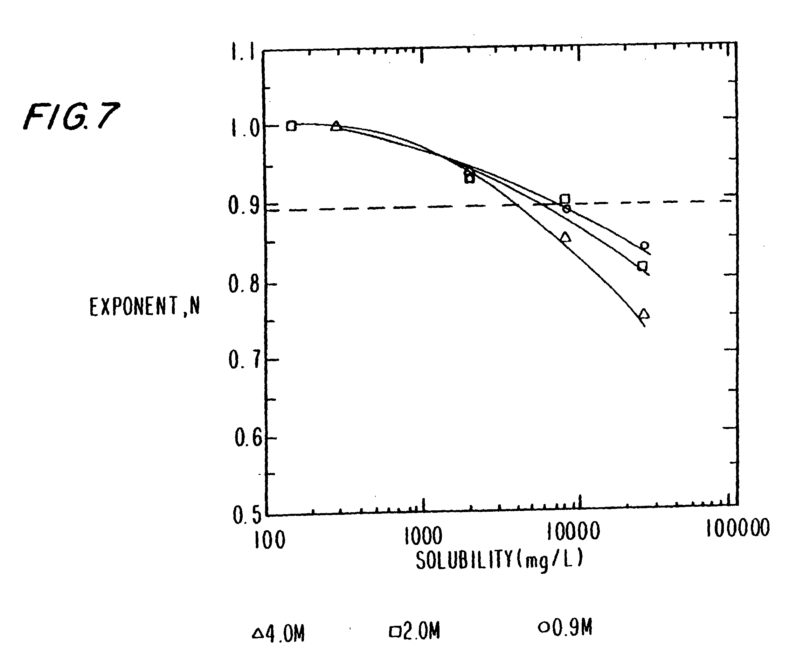

The change in release constant with solubility of the drug is shown in Figure 7. As the solubility of the drug increases, diffusion from the gel layer increases. Where the erosion of the gel layer is not fast enough to keep up with the diffusion, n decreases indicating non-constant release. As would be expected, this occurs more readily in higher molecular weight polymers.

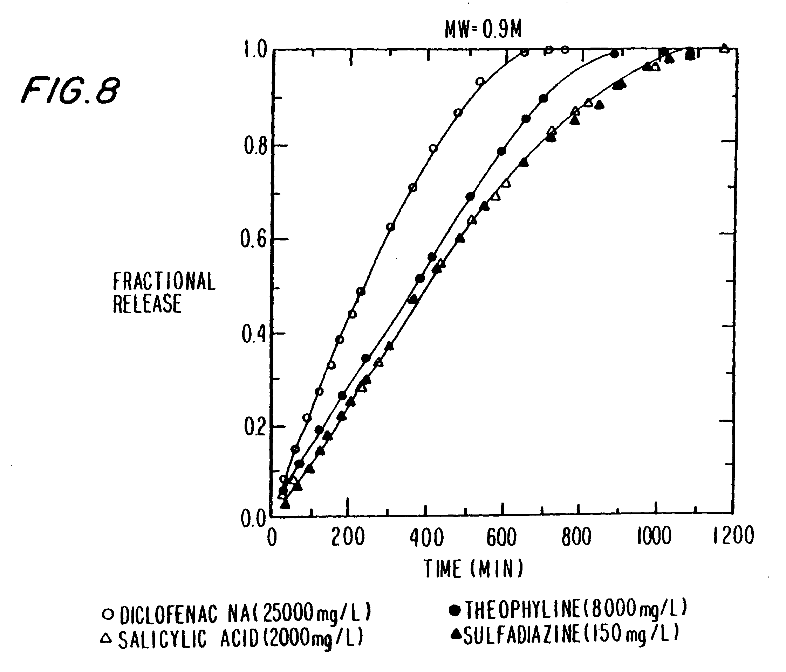

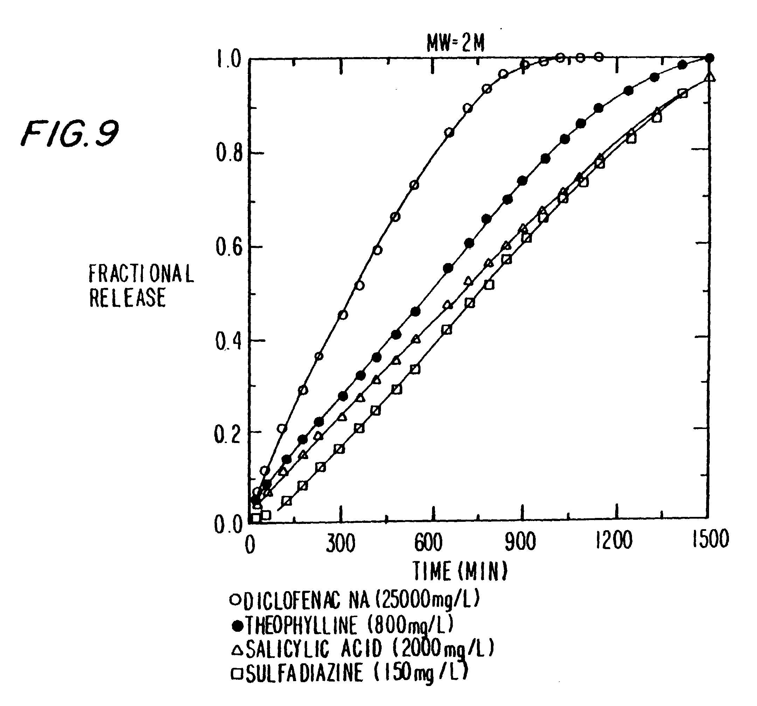

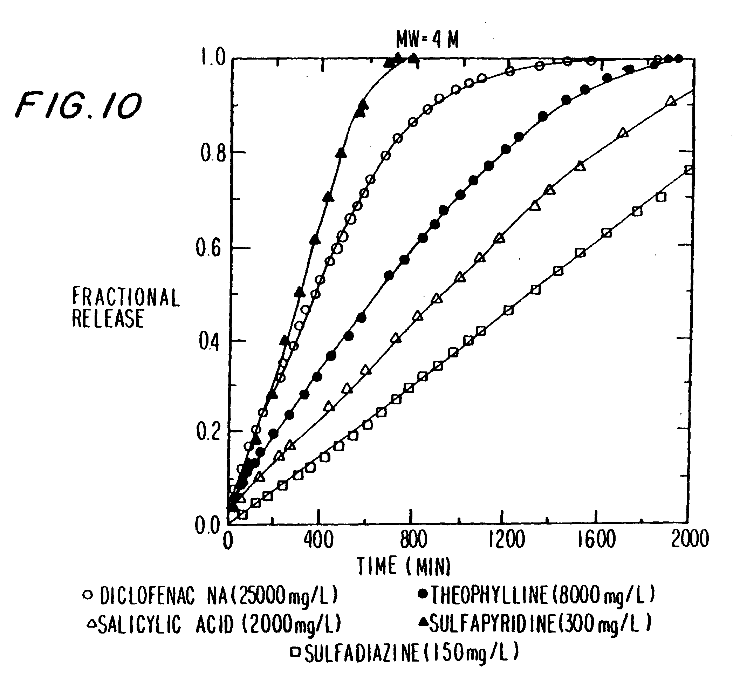

This effect is further demonstrated in Figures 8, 9, and 10. There, the particular drugs used for testing are shown along with their typical solubilities. The most constant release over the longest period of time is exhibited by sulfadiazine, the drug with the lowest solubility. The least constant release is exhibited by the more soluble drugs such as diclofenac NA and theophylline. The non-constant release worsens with higher molecular weight polymers.

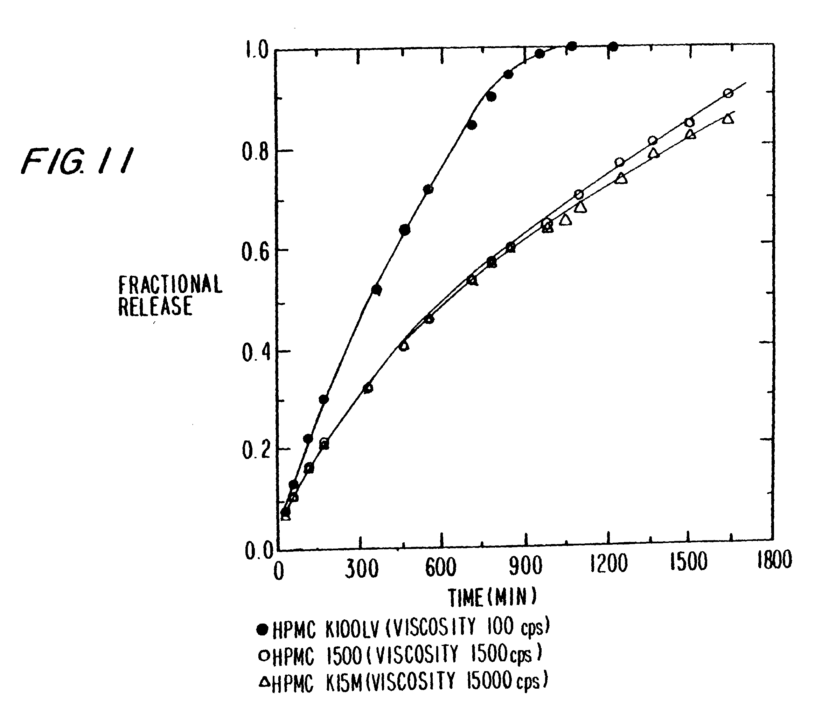

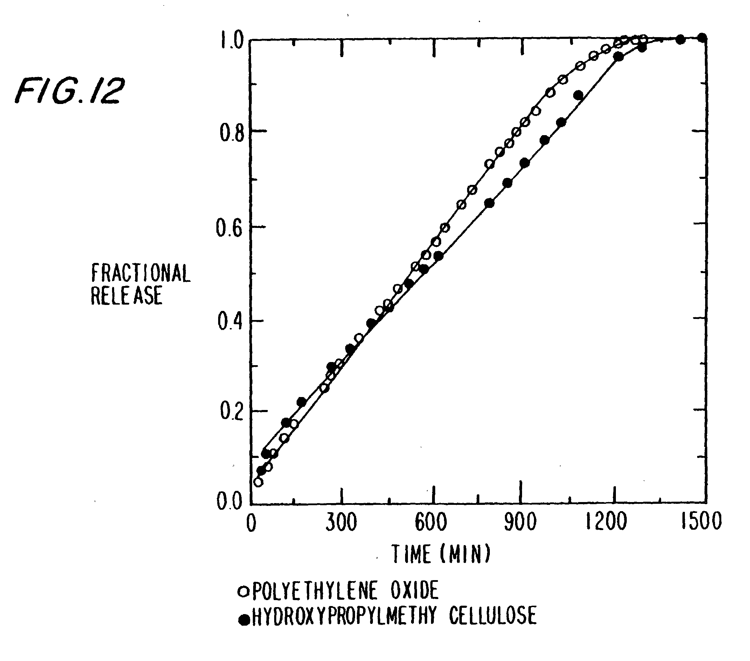

Figure 11 shows some linearity of drug delivery with HPMC, particularly where a low viscosity HPMC is used. HPMC having a viscosity of up to 100,000 centipoise can also be used with the present invention. However, as shown by Figure 12, the results achieved are improved substantially by the formation of a hole in the tablet. Forming a hole in the tablet allows the surface area of the tablet to increase as the tablet dissolves. This increases the amount of polymer which is exposed to the environment, and thus increases the amount of polymer which swells. This increases the dissolution rate of the polymer which makes the drug administration rate linear. This improves performance provided the hole in the tablet is not so small as to substantially disappear from the swelling which occurs when the tablet is first contacted with moisture.

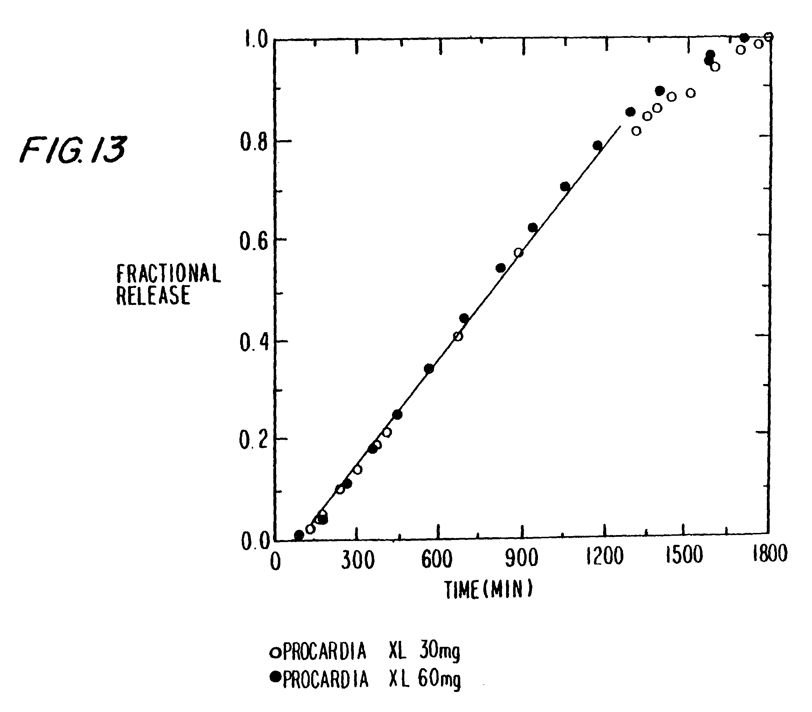

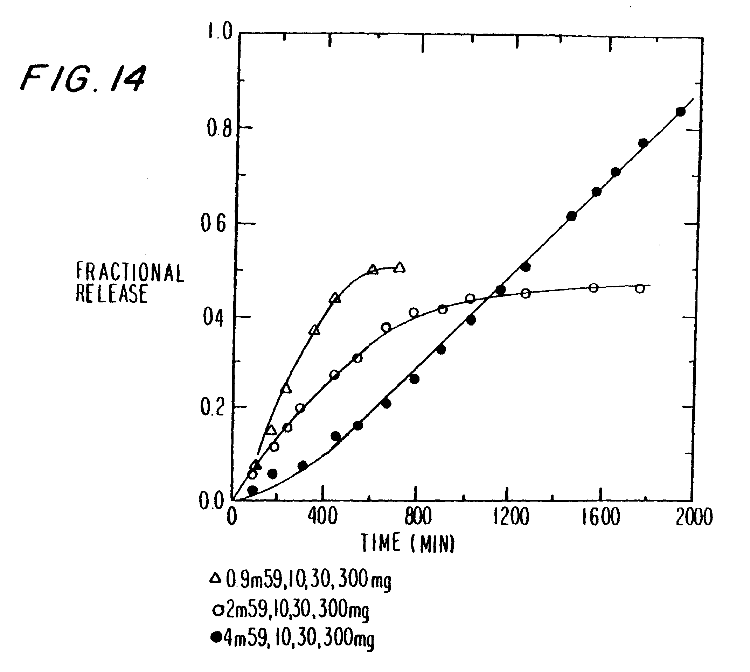

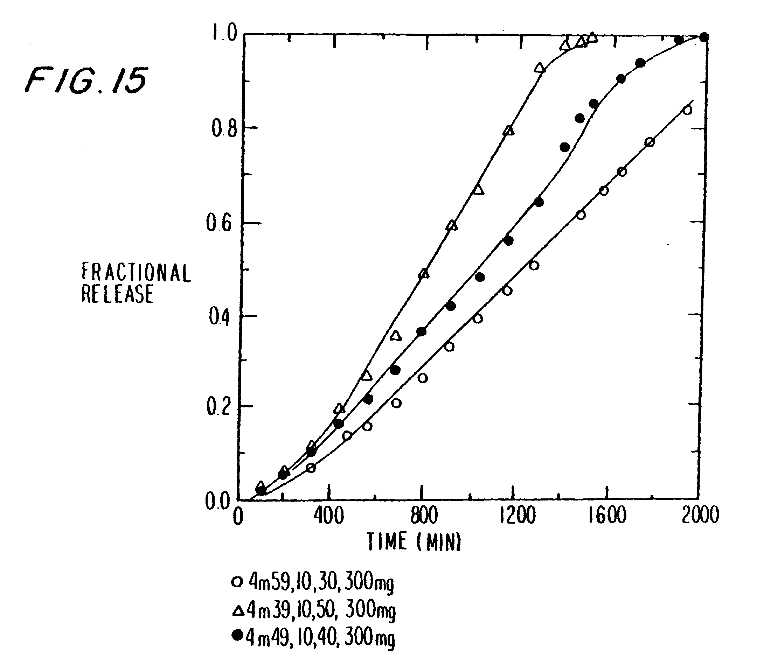

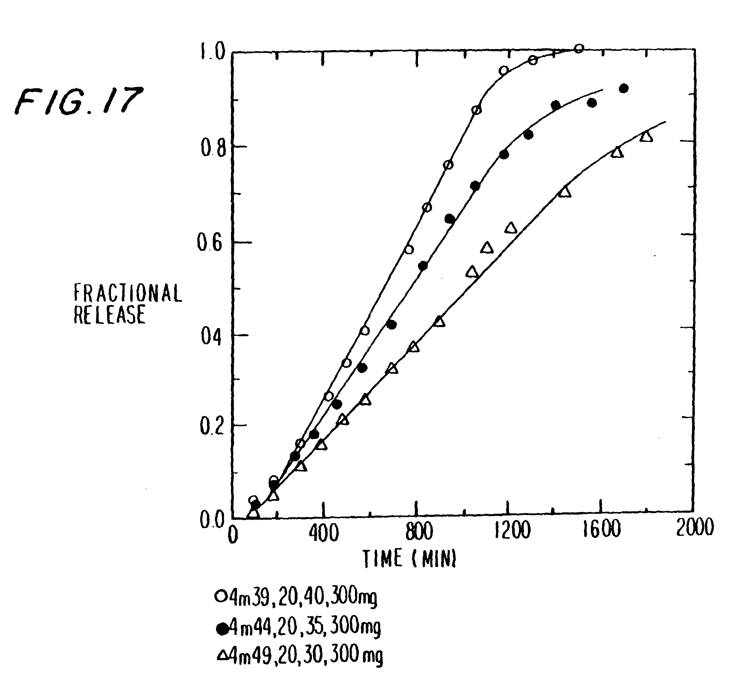

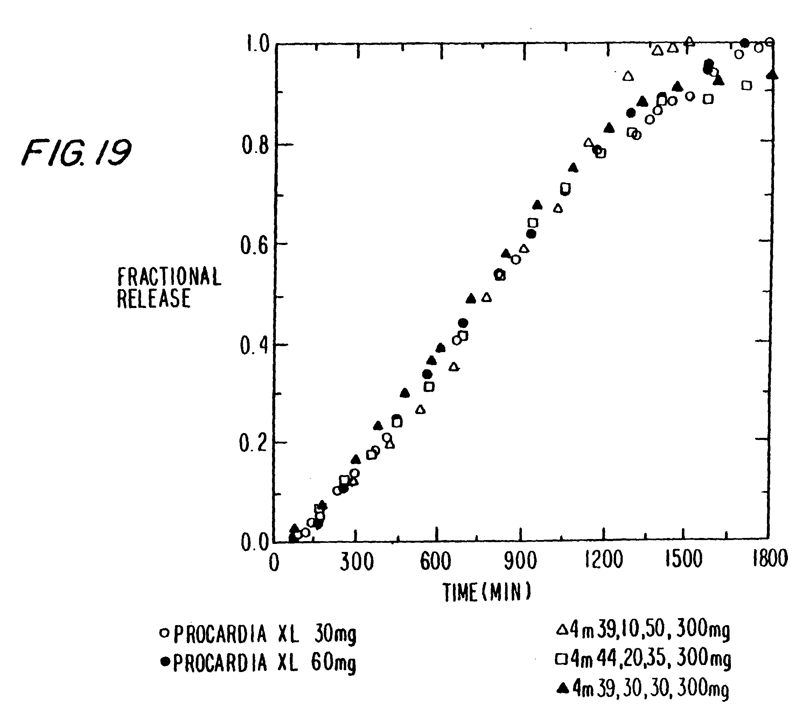

Figure 13 shows the release characteristics of Procardia XL nifedipine tablets of 30 and 60 milligrams (Pfizer, Inc., New York, NY). This figure may be compared to Figures 14 through 19 which show nifedipine tablets having various compositions made in accordance with the present invention. In each figure, the compositions of the tablets tested are shown as molecular weight of PEO (x $10^6$), percent of PEO, percent nifedipine, percent polyethylene glycol (PEG), and weight of tablet. Thus a tablet indicated to be, "0.9m59,10,30,300mg" has 59% PEO with a molecular weight of 0.9 x $10^6$, 10% nifedipine (or 30 mg), 30% PEG, in a 300 milligram tablet. The remaining 1% would comprise fillers and lubricants such as magnesium stearate.

In the tablets tested in Figures 14-19, PEG is used as a solubilizing agent to increase the solubility of the low solubility drug nifedipine. For the purposes of this application for calculating the composition of the excipient and the tablet as a whole, such a solubilizing agent is considered part of the drug, and not part of the excipient. This has been done since the use and proportion of the solubilizing agent is dependent on the particular drug used and the amount thereof. Other solubilizing agents useful in the present invention include cyclodextrins, hydroxypropylcyclodextrins, polyvinylpyrrolidone (PVP) and other solubilizing agents commonly employed in drug compounding.

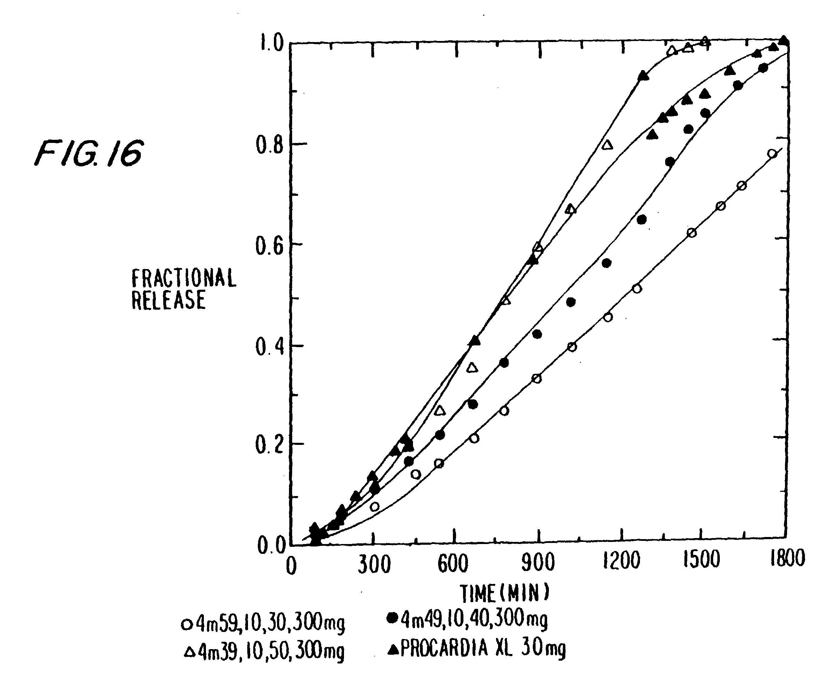

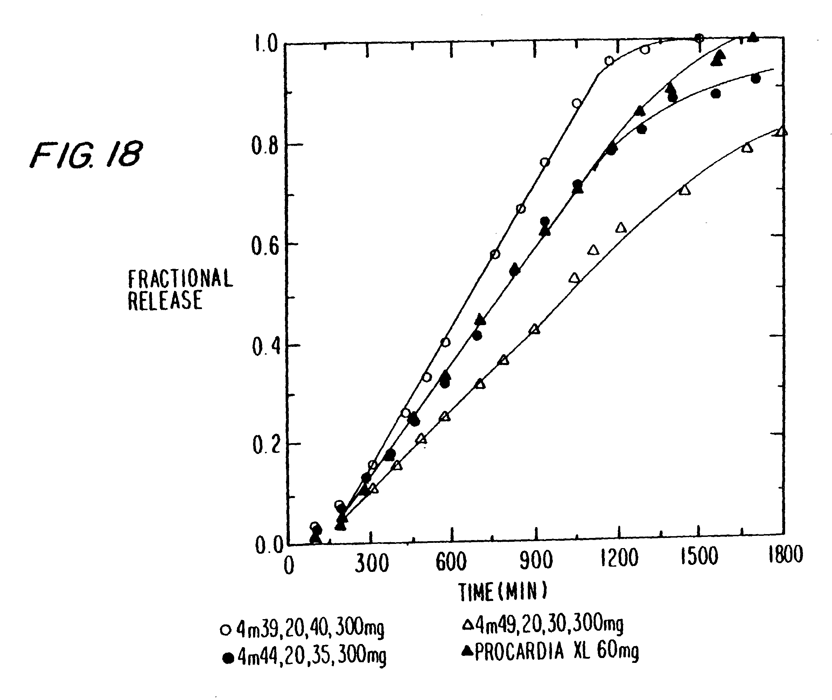

Figure 16 shows that a 300 mg PEO tablet having 40 to 50% PEG and 30 mg nifedipine performs very similarly to a 30 mg Procardia XL tablet. Figure 18 shows that a 300 mg PEO tablet having about 35% PEG and 60 mg nifedipine performs very similarly to a 60 mg Procardia XL tablet.

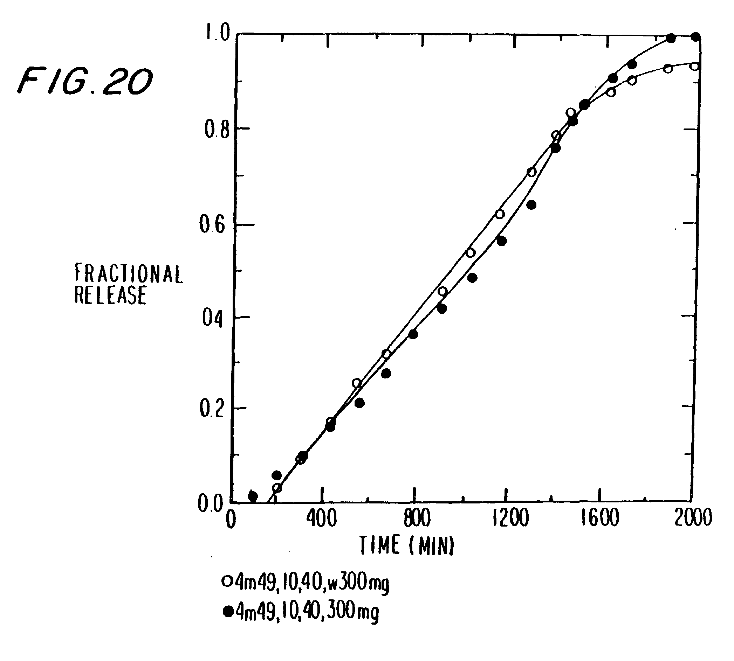

Figure 20 shows the fractional release of nifedipine from tablets of PEO having a molecular weight of $4x10^6$. The tablets were tested both in water (w 300 mg) and 0.54% sodiumlauryl sulfate, a common dissolution medium. The tablets were found to perform well.

## Claims

1. A controlled release tablet comprising a pharmaceutical agent and an excipient, wherein said excipient comprises:

   a) at least 50% of water swellable polymer; and
   b) a lubricant,

   wherein the water swellable polymer is polyethylene oxide, the swelling rate of said polymer is equal to the dissolution rate of said polymer when swollen, and tablet dissolution completion is coincident with pharmaceutical agent release completion.

2. A controlled release tablet comprising a pharmaceutical agent and an excipient, wherein said excipient comprises:

   polyethylene oxide and a lubricant; and

   wherein the pharmaceutical agent is released from the tablet at a rate expressed as $M_t/M_T=kt^n$ where:

t is time,

$M_t$ is the amount of the pharmaceutical agent which has been released at time t,

$M_T$ is the total amount of the pharmaceutical agent contained in the tablet,

k is a constant, and

n is the release kinetics exponent; and

    wherein the molecularweight and the solubility, respectively, of said polyethylene oxide and the pharmaceutical agent are selected such that n is about 0.89 to 1.0; and

    wherein tablet dissolution completion is coincident with pharmaceutical agent release completion.

**3.** A controlled release tablet according to claims 1 or 2 wherein said water swellable polymer is polyethylene oxide having an average molecular weight of 900,000 to 4,000,000.

**4.** A controlled release tablet according to claims 1 or 2 wherein said lubricant is magnesium stearate.

**5.** A controlled release tablet according to claims 1 or 2 wherein said lubricant is present in an amount up to 3% by weight of said excipient.

**6.** A controlled release tablet according to claims 1 or 2 wherein said excipient further comprises a diluent.

**7.** A controlled release tablet according to claim 6 wherein said diluent is selected from the group consisting of lactose, dextrose, sucrose, and calcium phosphate.

**8.** A controlled release tablet according to claims 6 or 7 wherein said diluent is present in an amount of up to 50% by weight of said excipient.

**9.** A controlled release tablet according to claims 1 or 2 further comprising a solubilizing agent.

**10.** A controlled release tablet according to claim 9 wherein said solubilizing agent is selected from the group consisting of polyethylene glycol, cyclodextrins, hydroxypropylcyclodextrins and polyvinylpyrrolidone (PVP).

**11.** A controlled release tablet according to claim 9 wherein said solubilizing agent is polyethylene glycol.

**12.** A controlled release tablet according to any of claims 9 to 11 wherein said solubilizing agent is present in an amount up to 150% of said excipient.

**13.** A controlled release tablet according to claims 1 or 2 wherein said pharmaceutical agent is present in an amount of from 10% to 40% by weight of said tablet.

**14.** A controlled release tablet according to claims 1 or 2 wherein said pharmaceutical agent is selected from the group consisting of nifedipine, diclofenac sodium, salicylic acid, theophylline, sulfapyridine, sulfadrazine, propranolol hydrochloride, and diltiazem hydrochloride.

**15.** A controlled release tablet according to claim 14 wherein said pharmaceutical agent is nifedipine.

**16.** A controlled release tablet according to claim 15 wherein said water swellable polymer is polyethylene oxide having an average molecular weight of 900,000 to 4,000,000.

**17.** A controlled release tablet according to claim 16 wherein said pharmaceutical agent is theophylline.

**18.** A controlled release tablet according to claim 14 wherein said pharmaceutical agent is diltiazem hydrochloride.

**19.** A controlled release tablet according to claim 18 wherein said water swellable polymer is polyethylene oxide having an average molecular weight of 900,000 to 4,000,000.

**20.** A controlled release tablet according to any of the preceding claims wherein said tablet has an aperture therethrough.

**21.** A controlled release tablet according to any of the preceding claims wherein the tablet exhibits zero order releasing kinetics for the pharmaceutical agent when the tablet is swollen.

**Patentansprüche**

**1.** Tablette zur kontrollierten Freisetzung, umfassend ein pharmazeutisches Mittel und einen Träger, wobei der Träger umfasst:

    a) mindestens 50% eines in Wasser quellbaren Polymers; und
    b) ein Gleitmittel,

worin das in Wasser quellbare Polymer Polyethylenoxid ist, wobei die Quellungsgeschwindigkeit des Polymers gleich der Lösungsgeschwindigkeit des gequollenen Polymers ist, und die vollständige Auflösung der Tablette mit der vollständigen Freisetzung des pharmazeutischen Mittels zusammenfällt.

2. Tablette zur kontrollierten Freisetzung eines pharmazeutischen Mittels und eines Trägers, worin der Träger umfasst,

    Polyethylenoxid und ein Gleitmittel; und

    worin das pharmazeutische Mittel aus der Tablette mit einer durch $M_t/M_T = kt^n$ ausgedrückten Geschwindigkeit freigesetzt wird, worin

    t die Zeit ist,
    $M_t$ die bis zur Zeit t freisetzte Menge an pharmazeutischem Mittel ist,
    $M_T$ die in der Tablette enthaltene Gesamtmenge an pharmazeutischem Mittel ist,
    k eine Konstante ist, und
    n der Exponent der Freisetzungskinetik ist; und

    wobei das Molekulargewicht und die Löslichkeit des Polyethylenoxids und des pharmazeutischen Mittel so gewählt sind, dass n etwa 0,89 bis 1,0 beträgt, und
    wobei die vollständige Auflösung der Tablette mit der vollständigen Freisetzung des pharmazeutischen Mittels zusammenfällt.

3. Tablette zur kontrollierten Freisetzung nach Anspruch 1 oder 2, wobei das in Wasser quellbare Polymer Polyethylenoxid mit einem durchschnittlichen Molekulargewicht von 900000 bis 4000000 ist.

4. Tablette zur kontrollierten Freisetzung nach Anspruch 1 oder 2, worin das Gleitmittel Magnesiumstearat ist.

5. Tablette zur kontrollierten Freisetzung nach Anspruch 1 oder 2, worin das Gleitmittel in einer Menge bis zu 3 Gew.-% des Trägers vorliegt.

6. Tablette zur kontrollierten Freisetzung nach Anspruch 1 oder 2, worin der Träger weiterhin ein Verdünnungsmittel umfasst.

7. Tablette zur kontrollierten Freisetzung nach Anspruch 6, worin das Verdünnungsmittel ausgewählt ist aus der Gruppe Laktose, Dextrose, Saccharose und Calciumphosphat.

8. Tablette zur kontrollierten Freisetzung nach Anspruch 6 oder 7, worin das Verdünnungsmittel in einer Menge von bis zu 50 Gew.-% des Trägers vorliegt.

9. Tablette zur kontrollierten Freisetzung nach Anspruch 1 oder 2, weiterhin umfassend einen Lösungsvermittler.

10. Tablette zur kontrollierten Freisetzung nach Anspruch 9, worin der Lösungsvermittler ausgewählt ist aus der Gruppe Polyethylenglykol, Cyclodextrine, Hydroxypropylcyclodextrine und Polyvinylpyrrolidon (PVP).

11. Tablette zur kontrollierten Freisetzung nach Anspruch 9, worin der Lösungsvermittler Polyethylenglykol ist.

12. Tablette zur kontrollierten Freisetzung nach einem der Ansprüche 9 bis 11, worin der Lösungsvermittler in einer Menge von bis zu 150% des Trägers vorliegt.

13. Tablette zur kontrollierten Freisetzung nach Anspruch 1 oder 2, worin das pharmazeutische Mittel in einer Menge von 10 % bis 40 % des Tablettengewichts vorliegt.

14. Tablette zur kontrollierten Freisetzung nach Anspruch 1 oder 2, worin das pharmazeutische Mittel ausgewählt ist aus Nifedipin, Diclofenac-Natrium, Salicylsäure, Theophyllin, Sulfapyridin, Sulfadrazin, Propanololhydrochlorid und Diltiazemhydrochlorid.

15. Tablette zur kontrollierten Freisetzung nach Anspruch 14, worin das pharmazeutische Mittel Nifedipin ist.

16. Tablette zur kontrollierten Freisetzung nach Anspruch 15, worin das in Wasser quellbare Polymer Polyethylenoxid mit einem durchschnittlichen Molekulargewicht von 900000 bis 4000000 ist.

17. Tablette zur kontrollierten Freisetzung nach Anspruch 16, worin das pharmazeutische Mittel Theophyllin ist.

18. Tablette zur kontrollierten Freisetzung nach Anspruch 14, worin das pharmazeutische Mittel Diltiazemhydrochlorid ist.

19. Tablette zur kontrollierten Freisetzung nach Anspruch 18, worin das in Wasser quellbare Polymer Polyethylenoxid mit einem durchschnittlichen Molekulargewicht von 900000 bis 4000000 ist.

20. Tablette zur kontrollierten Freisetzung nach einem der vorhergehenden Ansprüche, worin die Tablette eine durchgehende Öffnung aufweist.

21. Tablette zur kontrollierten Freisetzung nach einem der vorhergehenden Ansprüche, wobei die Tablette im gequollenen Zustand für das pharmazeutische Mittel eine Freisetzungskinetik nach nullter Ordnung aufweist.

**Revendications**

1. Un comprimé à libération contrôlée comprenant un agent pharmaceutique et un excipient, dans lequel ledit excipient comprend :

   a) au moins 50 % d'un polymère capable de gonfler à l'eau et
   b) un lubrifiant,

   dans lequel le polymère capable de gonfler à l'eau est un oxyde de polyéthylène, le taux de gonflement dudit polymère est égal au taux de dissolution dudit polymère lorsqu'il a gonflé et l'achèvement de la dissolution du comprimé coïncide avec l'achèvement de la libération de l'agent pharmaceutique.

2. Un comprimé à libération contrôlée comprenant un agent pharmaceutique et un excipient, dans lequel ledit excipient comprend :

   de l'oxyde de polyéthylène et un lubrifiant et

   dans lequel l'agent pharmaceutique est libéré du comprimé à un taux exprimé par $M_t/M_T = kt^n$ où :

   t est le temps,
   $M_t$ est la quantité de l'agent pharmaceutique qui a été libérée au temps t
   $M_T$ est la quantité totale de l'agent pharmaceutique contenue dans le comprimé,
   k est une constante et
   n est l'exposant de la cinétique de libération et

   dans lequel la masse moléculaire et la solubilité respectivement de l'oxyde de polyéthylène et de l'agent pharmaceutique sont sélectionnées de manière que n soit environ de 0,89 à 1,0 et
   dans lequel l'achèvement de la dissolution du comprimé coïncide avec l'achèvement de la libération de l'agent pharmaceutique.

3. Un comprimé à libération contrôlée selon les revendications 1 ou 2 dans lequel ledit polymère capable de gonfler à l'eau est un oxyde de polyéthylène ayant une masse moléculaire moyenne de 900 000 à 4 000 000.

4. Un comprimé à libération contrôlée selon les revendications 1 ou 2 dans lequel ledit lubrifiant est du stéarate de magnésium.

5. Un comprimé à libération contrôlée selon les revendications 1 ou 2 dans lequel ledit lubrifiant est présent en une quantité représentant jusqu'à 3 % en masse du dit excipient

6. Un comprimé à libération contrôlée selon les revendications 1 ou 2 dans lequel ledit excipient comprend en outre un diluant.

7. Un comprimé à libération contrôlée selon la revendication 6 dans lequel ledit diluant est sélectionné dans le groupe comprenant le lactose, le dextrose, le sucrose et le phosphate de calcium.

8. Un comprimé à libération contrôlée selon les revendications 6 ou 7 dans lequel ledit diluant est présent en une quantité représentant jusqu'à 50 % en masse du dit excipient.

9. Un comprimé à libération contrôlée selon les revendications 1 ou 2 comprenant en outre un agent solubilisant.

10. Un comprimé à libération contrôlée selon la revendication 9 dans lequel ledit agent solubilisant est sélectionné dans le groupe comprenant le polyéthylène glycol, les cyclodextrines, les hydroxypropylcyclodextrines et la polyvinylpyrrolidone (PVP).

11. Un comprimé à libération contrôlée selon la revendication 9 dans lequel ledit agent solubilisant est du polyéthylène glycol.

12. Un comprimé à libération contrôlée l'une quelconque des revendications 9 à 11 dans lequel ledit agent solubilisant est présent en une quantité pouvant atteindre 150 % dudit excipient.

13. Un comprimé à libération contrôlée selon les revendications 1 ou 2 dans lequel ledit agent pharmaceutique est présent en une quantité représentant de 10 à 40 % en masse dudit comprimé.

14. Un comprimé à libération contrôlée selon les revendications 1 ou 2 dans lequel ledit agent pharmaceutique est sélectionné dans le groupe comprenant la nifédipine, le diclofénac sodique, l'acide salicylique, la théophylline, la sulfapyridine, la sulfadrazine, le chlorhydrate de propranolol et le chlorhydrate de diltiazem.

15. Un comprimé à libération contrôlée selon la revendication 14 dans lequel ledit agent pharmaceutique est la nifédipine.

16. Un comprimé à libération contrôlée selon la revendication 15 dans lequel ledit polymère capable de gonfler à l'eau est un oxyde de polyéthylène ayant une masse moléculaire moyenne de 900 000 à 4 000 000.

17. Un comprimé à libération contrôlée selon la revendication 16 dans lequel ledit agent pharmaceutique est la théophylline.

**18.** Un comprimé à libération contrôlée selon la revendication 14 dans lequel ledit agent pharmaceutique est le chlorhydrate de diltiazem.

**19.** Un comprimé à libération contrôlée selon la revendication 18 dans lequel ledit polymère capable de gonfler à l'eau est un oxyde de polyéthylène ayant une masse moléculaire moyenne de 900 000 à 4 000 000.

**20.** Un comprimé à libération contrôlée selon l'une quelconque des revendications précédentes dans lequel ledit comprimé a une ouverture qui le traverse.

**21.** Un comprimé à libération contrôlée selon l'une quelconque des revendications précédentes dans lequel ledit comprimé exhibe une cinétique de libération d'ordre zéro de l'agent pharmaceutique lorsque le comprimé est gonflé.

FIG. 1

NORMALIZED GEL THICKNESS & DIAMETER

TIME (MIN)

MOLECULAR WEIGHT OF POLYMER

TABLET DIAMETER
● 0.9x10^6
▲ 2.0 x 10^6
■ 4.0 x 10^6

GEL THICKNESS
○ 0.9x10^6
△ 2.0x10^6
□ 4.0x10^6

FIG. 2

FIG.3

FRACTIONAL RELEASE

TIME(MIN)

○ pH=1.5
● pH=7.4
△ DISTILLED/DEIONIZED WATER

EP 0 821 582 B1

FIG.4

FRACTIONAL
RELEASE

TIME (MIN)

MOLECULAR WEIGHT OF POLYMER $0.9 \times 10^6$
  ○DILTIAZEM HYDROCHLORIDE 39 %
  ●DILTIAZEM HYDROCHLORIDE 20 %
  △THEOPHYLLINE 39%
  ▲THEOPHYLLINE 20%

EP 0 821 582 B1

## FIG.5

FRACTIONAL RELEASE

TIME (MIN)

MOLECULAR WEIGHT OF POLYMER 2.0x10$^6$
○ DILTIAZEM HYDROCHLORIDE 39%
● DILTIAZEM HYDROCHLORIDE 20%
△ THEOPHYLLINE 39%
▲ THEOPHYLLINE 20%

EP 0 821 582 B1

*FIG.6*

FRACTIONAL
RELEASE

TIME (MIN)

MOLECULAR WEIGHT OF POLYMER $0.9 \times 10^6$
o 50rpm        □ 150rpm         + MAGNETIC STIRRING
△ 100rpm       ● 200rpm

EP 0 821 582 B1

FIG.7

EXPONENT,N

SOLUBILITY(mg/L)

△4.0M     □2.0M     ○0.9M

EP 0 821 582 B1

FIG. 8

FIG. 9

FIG.10

FRACTIONAL RELEASE

TIME (MIN)

MW=4 M

o DICLOFENAC NA(25000 mg/L)    ●THEOPHYLLINE(8000 mg/L)
△SALICYLIC ACID (2000mg/L)    ▲SULFAPYRIDINE(300mg/L)
□SULFADIAZINE(150mg/L)

FIG. 11

FRACTIONAL
RELEASE

TIME (MIN)

● HPMC K100LV (VISCOSITY 100 cps)
○ HPMC 1500 (VISCOSITY 1500 cps)
△ HPMC K15M (VISCOSITY 15000 cps)

EP 0 821 582 B1

FIG. 12

FRACTIONAL RELEASE

TIME (MIN)

○ POLYETHYLENE OXIDE
● HYDROXYPROPYLMETHY CELLULOSE

EP 0 821 582 B1

FIG.13

FRACTIONAL RELEASE

TIME(MIN)

○PROCARDIA  XL  30mg
●PROCARDIA  XL  60mg

EP 0 821 582 B1

FIG. 14

△ 0.9m59,10,30,300 mg
o 2m59,10,30,300mg
● 4m59,10,30,300mg

FRACTIONAL RELEASE

TIME (MIN)

FIG.15

FRACTIONAL
RELEASE

TIME (MIN)

o 4m59,10,30,300mg
△4m39,10,50, 300mg
● 4m49,10,40, 300mg

EP 0 821 582 B1

FIG. 16

FRACTIONAL
RELEASE

TIME (MIN)

o 4m59,10,30,300mg      ● 4m49,10,40,300mg
△ 4m39,10,50,300mg      ▲ PROCARDIA XL 30mg

EP 0 821 582 B1

FIG.17

FRACTIONAL
RELEASE

TIME (MIN)

○4m39,20,40,300mg
●4m44,20,35,300mg
△4m49,20,30,300mg

EP 0 821 582 B1

FIG. 18

FIG.19

FRACTIONAL RELEASE

TIME (MIN)

△ 4m 39,10,50, 300mg
□ 4m 44,20,35, 300mg
▲ 4m 39,30,30, 300mg

○ PROCARDIA XL 30mg
● PROCARDIA XL 60mg

FIG. 20

FRACTIONAL
RELEASE

TIME (MIN)

○4m49,10,40,w300mg
●4m49,10,40,300mg

EP 0 821 582 B1